# EUROPÄISCHE PATENTSCHRIFT

(11) **EP 1 250 305 B1**
(45) Veröffentlichungstag und Bekanntmachung des Hinweises auf die Patenterteilung: **12.08.2009**
(21) Anmeldenummer: 01907445.9
(22) Anmeldetag: 12.01.2001
(51) Int. Cl.: C07C 51/09, C07C 51/44, C07C 51/48, C07C 53/02, B01J 14/00

(54) **ABWASSERREINIGUNG BEIM VERFAHREN ZUR HERSTELLUNG VON WASSERFREIER AMEISENSÄURE**
WASTE WATER TREATMENT IN A METHOD FOR PRODUCING FORMIC ACID WHICH IS FREE OF WATER
EPURATION DES EAUX RESIDUAIRES LORS DU PROCEDE DE PRODUCTION D'ACIDE FORMIQUE EXEMPT D'EAU

(30) Priorität: 24.01.2000 DE 10002794
(43) Veröffentlichungstag der Anmeldung: 23.10.2002
(73) Patentinhaber: BASF SE, 67056 Ludwigshafen (DE)
(72) Erfinder: AUER, Heinz, 68809 Neulussheim (DE); BESSLING, Bernd, 67269 Grünstadt (DE); HAMMER, Hans, 68219 Mannheim (DE); HASSE, Hans, 67661 Kaiserslautern (DE); SAUER, Friedrich, 67271 Obersülzen (DE); VICARI, Maximilian, 67117 Limburgerhof (DE); ADRIAN, Till, 67240 Bobenheim-Roxheim (DE)
(74) Vertreter: Isenbruck, Günter
(86) Internationale Anmeldenummer: PCT/EP2001/000346
(87) Internationale Veröffentlichungsnummer: WO 2001/055069

(56) Entgegenhaltungen:
- EP-A- 0 017 866
- DATABASE WPI Section Ch, Week 199523 Derwent Publications Ltd., London, GB; Class A41, AN 1995-172870 XP002175587 & JP 07 082191 A (MITSUBISHI KASEI CORP), 28. März 1995 (1995-03-28)

## Beschreibung

Die Erfindung betrifft eine Vorrichtung und ein Verfahren zur Gewinnung von wasserfreier oder weitgehend wasserfreier Ameisensäure und die Verwendung eines Wasserdampfstroms in diesem Verfahren.

Aus "Ullmanns Encyklopädie der technischen Chemie", 4. Auflage, Band 7, Seite 365, ist bekannt, Ameisensäure durch Acydolyse von Formamid mit Schwefelsäure herzustellen. Dieses Verfahren hat jedoch den Nachteil, daß stöchiometrische Mengen Ammoniumsulfat als Zwangsanfall erhalten werden.

Ein weitere Möglichkeit zur Herstellung von Ameisensäure besteht in der Hydrolyse von Methylformiat, das aus Methanol und Kohlenmonoxid synthetisiert wird. Dabei liegen die folgenden Gleichungen zugrunde:

| | | | |
|---|---|---|---|
| | CO + CH₃-OH | → | CH₃-O-CO-H |
| | CH₃O-CO-H + H₂O | → | CH₃-OH + H-CO-OH |
| Brutto: | CO + H₂O | → | H-CO-OH |

Die in "Ullmanns Encyklopädie der technischen Chemie", 4. Auflage, Band 7, Seite 366 beschriebene Hydrolyse von Methylformiat

HCOOCH₃ + H₂O HCOOH + CH₃OH

hat den Nachteil einer ungünstigen Lage des Hydrolysegleichgewichtes. Eine Gleichgewichtsverschiebung durch destillative Entfernung der gewünschten Verfahrensprodukte ist nicht möglich, da Methylformiat (Sdp. 32°C) wesentlich niedriger als Methanol (Sdp. 65°C) und Ameisensäure (Sdp. 101°C) siedet. Aus der anfallenden wäßrigen Ameisensäurelösung kann wasserfreie Ameisensäure wegen Azeotropbildung mit Wasser nicht ohne weiteres destillativ gewonnen werden. Die Schwierigkeit besteht also darin, aus dem Hydrolysegemisch des Methylformiats wasserfreie Ameisensäure zu gewinnen.

Ein in der EP-B-0 017 866 beschriebenes, die Verfahrensschritte a) bis g) aufweisendes Verfahren ermöglicht ausgehend von Methylformiat die Herstellung wasserfreier Ameisensäure. Dabei erhält man wasserfreie Ameisensäure, falls man
a) Methylformiat der Hydrolyse unterwirft,
b) vom erhaltenen Hydrolysegemisch Methanol sowie überschüssiges Methylformiat abdestilliert,
c) das Ameisensäure und Wasser enthaltende Sumpfprodukt der Destillation (b) in einer Flüssig-flüssig-Extraktion mit einem hauptsächlich die Ameisensäure aufnehmenden Extraktionsmittel extrahiert,
d) die hierbei erhaltene Ameisensäure, Extraktionsmittel und eine Teilmenge des Wassers aufweisende Extraktphase einer Destillation unterwirft,
e) das bei dieser Destillation erhaltene Wasser und eine Teilmenge der Ameisensäure enthaltende Kopfprodukt in den unteren Teil der Destillationskolonne der Stufe (b) zurückführt,
f) das vorwiegend Extraktionsmittel und Ameisensäure enthaltende Sumpfprodukt der Destillationsstufe (d) destillativ in wasserfreie Ameisensäure und das Extraktionsmittel auftrennt und
g) das die Stufe (f) verlassende Extraktionsmittel in den Verfahrensgang zurückführt.

Es ist bei diesem Verfahren besonders zweckmäßig,
h) die Destillationsschritte (b) und (d) in einer einzigen Kolonne vorzunehmen,
i) das für die Hydrolyse benötigte Wasser dampfförmig in den unteren Teil der für die Durchführung von Schritt (b) vorgesehenen Kolonne einzubringen,
k) Methylformiat und Wasser bei der Hydrolyse (a) im Molverhältnis 1:2 bis 1:10 einzusetzen und/oder
1) als Extraktionsmittel ein Carbonsäureamid der allgemeinen Formel I 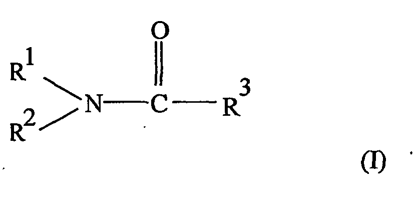 einzusetzen, in der die Reste R¹ und R² Alkyl-, Cycloalkyl-, Aryl- oder Aralkylgruppen bedeuten oder R¹ und R² gemeinsam zusammen mit dem N-Atom einen heterocyclischen 5- oder 6-Ring ausbilden und daß nur einer der Reste eine Arylgruppe ist und in der R³ für Wasserstoff oder eine C₁-C₄-Alkylgruppe steht.

Im folgenden werden die Verfahrensschritte (a) bis (i) des vorstehend beschriebenen Verfahrens näher erläutert.

### Verfahrensschritt (a)

Die Hydrolyse wird üblicherweise in einem Temperaturbereich von 80 bis 150°C durchgeführt.

### Verfahrensschritt (b)

Die Destillation des Hydrolysegemisches kann prinzipiell bei beliebigem Druck, bevorzugt 0,5 bis 2 bar, vorgenommen werden. Im allgemeinen empfiehlt sich das Arbeiten unter Normaldruck. In diesem Fall beträgt die Temperatur im Kolonnensumpf etwa 110°C und am Kolonnenkopf etwa 30 bis 40°C. Das Hydrolysegemisch wird zweckmäßigerweise in einem Temperaturbereich von 80 bis 150°C zugegeben, und das Methanol entnimmt man vorzugsweise flüssig bei Temperaturen von 55 bis 65°C. Eine zufriedenstellende Trennung des Gemisches in Methylformiat sowie Methanol einerseits und die wäßrige Ameisenäure andererseits ist bereits mit einer Destillationskolonne möglich, die 25 theoretische Böden aufweist (bevorzugt ist eine theoretische Bodenzahl von 35 bis 45). Die Bauart der für den Verfahrensschritt (b) vorgesehenen Kolonne kann beliebig sein, besonders empfiehlt sich jedoch eine Siebboden- oder Füllkörper-Kolonne.

### Verfahrensschritt (c)

Die Flüssig-flüssig-Extraktion der Ameisensäure aus ihrer wäßrigen Lösung mittels eines Extraktionsmittels wird vorzugsweise bei Normaldruck und bei Temperaturen von 60 bis 120, insbesondere 70 bis 90°C im Gegenstrom vorgenommen. Je nach Art des Extraktionsmittels benötigt man in der Regel Extraktionseinrichtungen mit 1 bis 12 theoretischen Trennstufen. Geeignete Extraktionseinrichtungen dafür sind insbesondere Flüssig-flüssig-Extraktionskolonnen. In den meisten Fällen erzielt man mit 4 bis 6 theoretischen Trennstufen befriedigende Ergebnisse.

Die Wahl des Extraktionsmittels ist nicht beschränkt. Als Extraktionsmittel besonders geeignet sind Carbonsäureamide der vorstehend genannten allgemeinen Formel I. Derartige Extraktionsmittel sind vor allem N,N-Di-n-butylformamid sowie außerdem N,N-Di-n-butylacetamid, N-Methyl-N-2-heptylformamid, N-n-Butyl-N-2-ethylhexylformamid, N-n-Butyl-N-cyclohexylformamid und N-Ethylformanilid sowie Gemische dieser Verbindungen. Weitere geeignete Extraktionsmittel sind u.a. Diisopropylether, Methylisobutylketon, Ethylacetat, Tributylphosphat und Butandiolformiat.

### Verfahrensschritt (d)

Die Extraktphase wird in einer entsprechenden Destillationseinrichtung destillativ in eine Flüssigphase, die in der Regel vorwiegend Ameisensäure und Extraktionsmittel aufweist, sowie eine vorwiegend Wasser und geringe Mengen Ameisensäure aufweisende Dampfphase getrennt. Es handelt sich dabei um eine Extraktivdestillation. Die Sumpftemperatur beträgt vorzugsweise 140 bis 180°C. Ein ausreichender Trenneffekt wird in der Regel ab 5 theoretischen Böden erzielt.

### Verfahrensschritt (e)

Die Rückführung des Ameisensäure-Wassergemischs erfolgt in der Regel dampfförmig.

### Verfahrensschritte (f) und (g)

Die Destillationseinrichtung (meist als Kolonne ausgebildet) zur Durchführung der Stufe (f) wird zweckmäßigerweise unter vermindertem Druck - etwa 50 bis 300 mbar - und entsprechend niedrigen Kopftemperaturen - etwa 30 bis 60°C - betrieben.

### Verfahrensschritt (h)

Diese Variante des Verfahrens betrifft die Schritte (b) und (d). Die Destillationseinrichtungen zur Durchführung der Stufen b) und d) werden in einer Gesamtdestillationseinrichtung angeordenet. Die Destillationseinrichtungen sind dabei in der Regel als Kolonnen ausgebildet.

### Verfahrensschritt (i)

In diesem Schritt wird für die Hydrolyse benötigtes Wasser in Form von Wasserdampf bereitgestellt.

Es hat sich als besonders vorteilhaft herausgestellt, für die Hydrolyse benötigtes Wasser dampfförmig in den unteren Teil der für die Durchführung der Stufe b) vorgesehenen Destillationseinrichtung einzubringen. Dadurch wird mit dem ohnehin für die Hydrolyse benötigten Wasser gleichzeitig noch Wärmeenergie in Form von Wasserdampf in das Verfahren eingeführt. Durch die Einführung des Wasserdampfes wird soviel Energie in die Destillationseinrichtung eingebracht, daß der entsprechende Sumpfverdampfer entsprechend kleiner ausgelegt werden kann, da durch den Sumpfverdampfer weniger Energie eingebracht werden muß.

Es ist notwendig, einen Teil der wäßrigen Phase, die bei der Flüssig-flüssig-Extraktion der Ameisensäure entsteht, aus dem Verfahren auszuschleusen, damit ein Auslaß für schwerflüchtige Nebenkomponenten (zum Beispiel Salze) geschaffen wird. Anderenfalls würden sich schwerflüchtige Nebenkomponenten im Prozeß anreichern, was sich negativ auf die Produktqualität auswirkt. In der aus der Extraktionseinrichtung zur Durchführung der Flüssig-flüssig-Extraktion ablaufenden wäßrigen Phase sind jedoch noch geringe Mengen an Wertstoffen, insbesondere Ameisensäure und Extraktionsmittel enthalten. Diese Stoffe dürfen nicht in das Abwasser gelangen, da die eingesetzten Extraktionsmittel in der Regel biologisch schwer abbaubar sind. Um das anfallende Abwasser zu reinigen und die Wertstoffe zurückzugewinnen, wird allgemein eine Mehrsäulen-Adsorber-Verschaltung verwendet, deren Anschaffung und Betrieb sehr kostspielig ist. Besonders aufwendig ist in der Regel die Regenerierung des Adsorbens. Als Adsorbens wird vorwiegend Aktivkohle eingesetzt.

Aufgabe der vorliegenden Erfindung ist es, ein Verfahren bereitzustellen, durch das die Wiedergewinnung der Wertstoffe aus dem die Extraktionseinrichtung verlassenden Abwasser gewährleistet wird. Dabei soll das Abwasser so gründlich von den Wertstoffen und gegebenenfalls von anderen Verunreinigungen befreit werden, daß eine weitere Reinigung des Abwassers nicht mehr erforderlich ist.
Das Verfahren soll praktisch durchführbar und wirtschaftlich attraktiv sein.

Die Lösung dieser Aufgabe geht aus von dem Verfahren zur Gewinnung von wasserfreier oder weitgehend wasserfreier Ameisensäure, bei dem man
(i) Methylformiat der Hydrolyse unterwirft,
(ii) vom erhaltenen Hydrolysegemisch Methanol sowie überschüssiges Methylformiat in einer Destillationseinrichtung abdestilliert und in den unteren Teil dieser Destillationseinrichtung Wasserdampf einleitet,
(iii) das Ameisensäure und Wasser enthaltende Sumpfprodukt der Destillation (ii) in einer Flüssig-flüssig-Extraktion mit einem hauptsächlich die Ameisensäure aufnehmenden Extraktionsmittel extrahiert, und eine Teilmenge der bei der Flüssig-flüssig-Extraktion entstehenden wäßrigen Phase als Abwasser aus dem Prozeß ausschleust,
(iv) die hierbei erhaltene, Ameisensäure, Extraktionsmittel und eine Teilmenge des Wassers aufweisende Extraktphase einer Destillation unterwirft,
(v) das bei dieser Destillation erhaltene, Wasser und eine Teilmenge der Ameisensäure enthaltende Kopfprodukt in den unteren Teil der Destillationseinrichtung der Stufe (ii) zurückführt,
(vi) das vorwiegend Extraktionsmittel und Ameisensäure enthaltende Sumpfprodukt der Destillationsstufe (iv) destillativ in wasserfreie oder weitgehend wasserfreie Ameisensäure und das Extraktionsmittel auftrennt und
(vii) das die Stufe (vi) verlassende Extraktionsmittel in den Verfahrensgang zurückführt.

Das erfindungsgemäße Verfahren ist dadurch gekennzeichnet, daß Wasserdampf, der in die für die Durchführung der Stufe (ii) vorgesehene Destillationseinrichtung eingeleitet wird, vor dem Einleiten in diese Destillationseinrichtung als Strippdampf für eine Abwasserstrippung des in Stufe (iii) aus dem Prozeß ausgeschleusten Abwassers eingesetzt wird.

Unter weitgehend wasserfreier Ameisensäure soll Ameisensäure verstanden werden, die maximal 30 %, bevorzugt maximal 15 % Wasser, enthält. Unter Wasserdampf soll gasförmiges Wasser verstanden werden, wobei letzteres auch noch flüssige Bestandteile und andere Komponenten (außer Wasser) enthalten kann.

Das erfindungsgemäße Verfahren ist besonders wirtschaftlich, da der ohnehin für das Verfahren benötigte Wasserdampf zusätzlich noch für die Abwasseraufbereitung eingesetzt wird. Das Verfahren ist energiesparend, erfordert einen geringen Investitionsaufwand und ist einfach durchführbar. Das Abwasser kann durch das Verfahren so effektiv gereinigt werden, daß das Abwasser anschließend direkt in eine Kläranlage eingeleitet werden kann. Außerdem werden Wertstoffe des Verfahrens wiedergewonnen und rezykliert.

In einer bevorzugten Ausführungsform der Erfindung wird als Extraktionsmittel ein Carbonsäureamid der allgemeinen Formel I 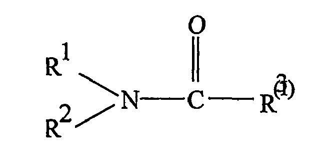 eingesetzt, in der die Reste R¹ und R² Alkyl-, Cycloalkyl-, Aryl- oder Aralkylgruppen bedeuten oder R¹ und R² gemeinsam zusammen mit dem N-Atom einen heterocyclischen 5- oder 6-Ring ausbilden und daß nur einer der Reste eine Arylgruppe ist und in der R³ für Wasserstoff oder eine C₁-C₄-Alkylgruppe steht. Besonders bevorzugte Extraktionsmittel sind N,N-Di-n-butylformamid, N,N-Din-butylacetamid, N-Methyl-N-2-heptylformamid, N-n-Butyl-N-2-ethylhexylformamid, N-n-Butyl-N-cyclohexylformamid und/oder N-Ethylformanilid. Als Extraktionsmittel kann ein einziges Extraktionsmittel oder ein Extraktionsmittelgemisch eingesetzt werden.

In einer bevorzugten Ausführungsform der Erfindung werden die Schritte (ii) und (iv) in einer einzigen Destillationseinrichtung durchgeführt.

Der als Strippdampf eingesetzte Wasserdampf weist in der Regel eine Temperatur von 110 bis 200°C, bevorzugt von 140° bis 180°C, auf. Die Temperatur, mit der der Strippdampf in die Destillationseinrichtung eingeleitet wird, ist meist niedriger.

Die Erfindung betrifft auch die Verwendung eines Wasserdampfstroms in dem vorstehend beschriebenen Verfahren. Dabei wird der Wasserdampfstrom zunächst als Strippdampfstrom für Abwasserstrippung und anschließend als Eduktstrom zur Bereitstellung von Wasser für die Hydrolyse von Methylformiat verwendet.

Erfindungsgemäß wird auch eine Vorrichtung zur Durchführung des vorstehend beschriebenen Verfahrens bereitgestellt. Diese enthält,
α) einen Synthesereaktor,
β) einen Hydrolysereaktor
χ) eine Destillationseinrichtung zur Durchführung der Stufe (ii),
δ) eine Destillationseinrichtung zur Durchführung der Stufe (iv),
ε) eine Extraktionseinrichtung
φ) eine Destillationseinrichtung zur Durchführung der Stufe (vi) und
γ) eine Abwasserstrippeinrichtung.

Als Synthesereaktor soll eine Einrichtung verstanden werden, in der einerseits die Synthese von Methylformiat erfolgt (meist in einem entsprechenden Reaktor) und in der gegebenenfalls andererseits noch eine Trennung des erhaltenen Hydrolysegemischs (meist in einer dem Reaktor nachgeschalteten Destillationseinrichtung) durchgeführt wird. Der Hydrolysereaktor, in dem die Hydrolyse von Methylformiat erfolgt, kann beliebig ausgebildet sein. Als Destillationseinrichtungen werden bevorzugt Destillationskolonnen eingesetzt. Die Extraktionseinrichtung ist in der Regel als Flüssig-flüssig-Extraktionskolonne ausgebildet. Als Abwasserstrippeinrichtung wird in der Regel eine Strippkolonne eingesetzt. Meist weist diese Strippkolonne 3 bis 20, bevorzugt 5 bis 10, thermodynamische Trennstufen auf.

In einer bevorzugten Ausführungsform der Erfindung ist die Destillationseinrichtung zur Durchführung der Stufe (ii) und die Destillationseinrichtung zur Durchführung der Stufe (iv) in einer einzigen Destillationseinrichtung angeordnet. Letztere ist in der Regel als Destillationskolonne ausgebildet.

### Die anliegende Zeichnung zeigt

- in Fig. 1 sowie in Fig. 2: Schemata für Anlagen für die Durchführung von Verfahren zur Herstellung von wasserfreier Ameisensäure nach dem Stand der Technik und
- in Fig. 3 sowie in Fig. 4: Schemata von Anlagen zur Durchführung des erfindungsgemäßen Verfahrens.

Die über, neben bzw. unter den Verbindungsleitungen eingetragenen Zahlen sollen ein Hinweis auf die in den entsprechenden Strömen enthaltenen Komponenten geben, die den größten Anteil haben. Da diese Anteile jedoch variieren können, können diese Bezugszeichen nur als Richtwert dienen. Dabei bedeuten 21 Methylformiat, 22 Wasser, 23 Ameisensäure, 24 Methanol, 25 Extraktionsmittel, 26 Abwasser und 27 Kohlenmonoxid.

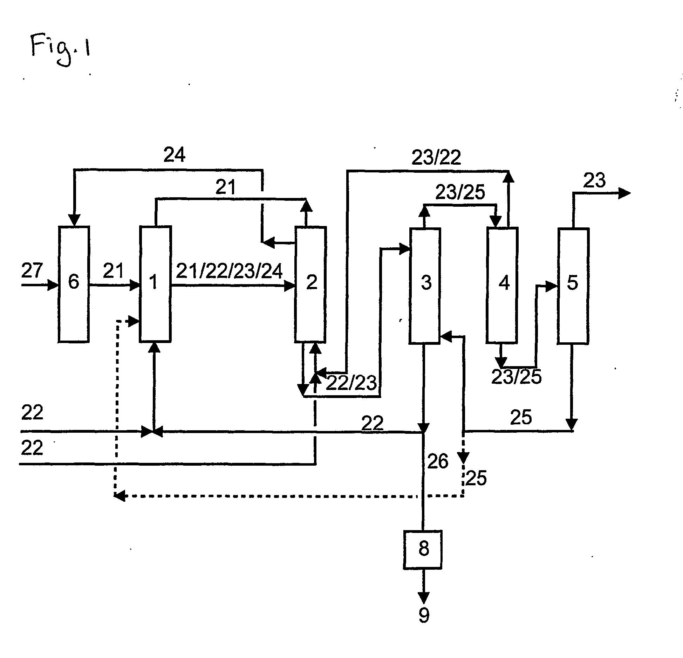

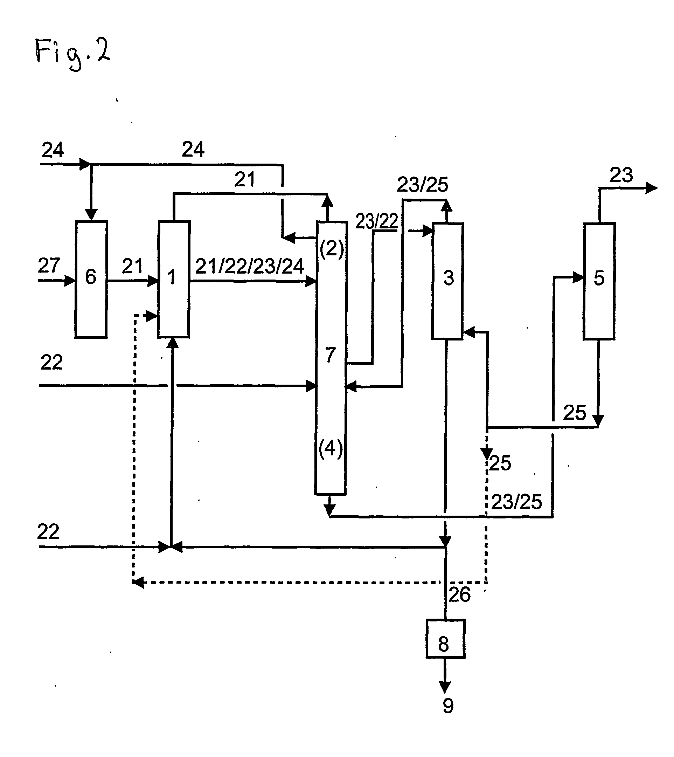

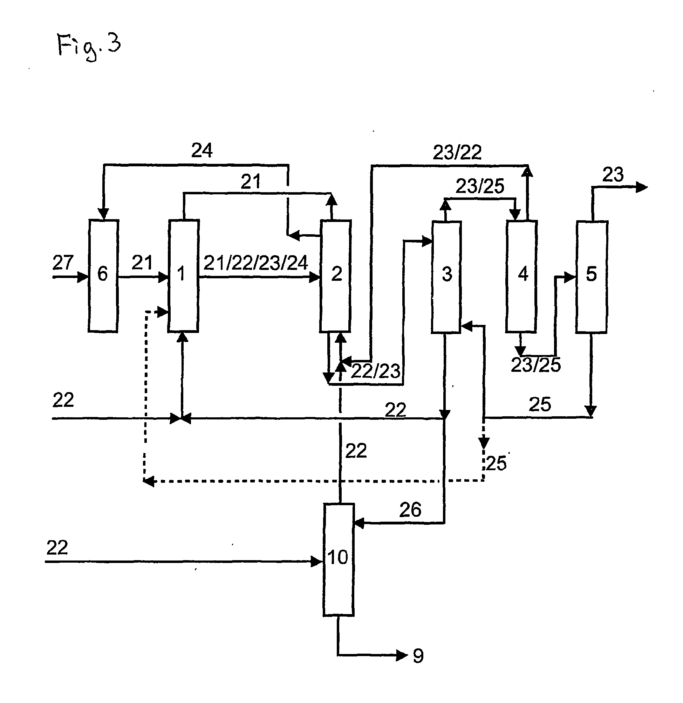

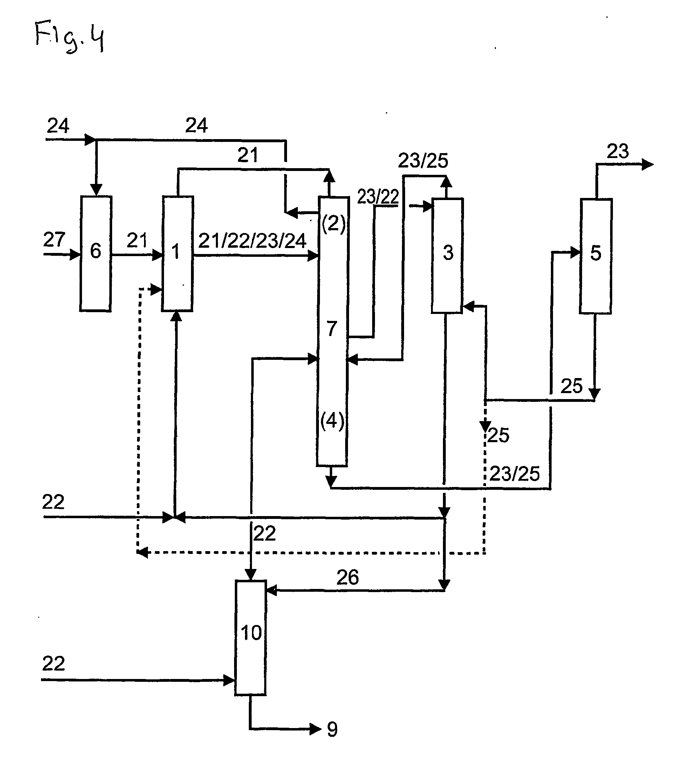

Die in Fig. 1 bzw. Fig. 2 aufgezeigten Anlagen zur Durchführung eines Verfahrens nach dem Stand der Technik und die in Fig. 3 bzw. Fig. 4 dargestellten Anlagen zur Durchführung des erfindungsgemäßen Verfahrens haben gemeinsam, daß diese einen Synthesereaktor 6, einen Hydrolysereaktor 1, eine Destillationseinrichtung 2 zur Durchführung der Stufe (ii), eine Destillationseinrichtung 4 zur Durchführung der Stufe (iv), eine Extraktionseinrichtung 3 und eine Destillationseinrichtung 5 zur Durchführung der Stufe (vi) aufweisen. Dabei können die Destillationseinrichtungen 2, 4 in einer gemeinsamen Destillationseinrichtung 7 angeordnet sein.

Die Anlagen zur Durchführung des erfindungsgemäßen Verfahrens (Fig. 3 und Fig. 4) enthalten im Gegensatz zu den in Fig. 1 bzw. Fig. 2 aufgezeigten Anlagen eine Abwasserstrippeinrichtung 10. Das gereinigte Abwasser wird aus dieser in die Kläranlage 9 geleitet. Die in Fig. 1 bzw. Fig. 2 aufgezeigten Anlagen zur Durchführung des Verfahrens nach dem Stand der Technik enthalten anstatt der Abwasserstrippeinrichtung 10 eine Adsorbereinrichtung 8.

Im folgenden soll die Erfindung anhand eines Ausführungsbeispiels näher erläutert werden.

### Beispiel

Der Beispielsversuch wird in einer Anlage durchgeführt, die schematisch in Fig. 4 aufgezeigt ist. Als Extraktionsmittel wird N,N-Di-n-butylformamid eingesetzt. Es werden kontinuierlich 5,3 kg wäßrige Ameisensäure hergestellt. Dabei fallen ca. 400 g/h Abwasser an. Das Abwasser wird in einer Strippkolonne mit ca. 1 kg/h Dampf gestrippt. Das Extraktionsmittel wird dabei vollständig aus dem Abwasser entfernt. Die zur Strippung verwendete Technikumskolonne hat einen Durchmesser von 30 mm und ist mit 30 Glockenböden ausgestattet. Die Ergebnisse des Versuchs sind in der nachstehenden Tabelle 1 aufgeführt.

**Tabelle 1**

| | **Abwasser (ungereinigt)** | **Abwasser (gereinigt)** | **Strippdampf (unbeladen)** | **Strippdampf (beladen)** |
|---|---|---|---|---|
| Anfall im Verfahren kg/h | 0,375 | 0,385 | 1,052 | 1,042 |
| wasser Anteil Gew.-% | 98,25 | 99,86 | 100 | 99,42 |
| Extraktionsmittel Anteil Gew.-% | 0,76 | 0 | 0 | 0,27 |
| Ameisensäure Anteil Gew.-% | 0,99 | 0,14 | 0 | 0,30 |
| Temperatur °C | 110 | 115 | 160 | 114 |

Tabelle 1 zeigt, daß durch das erfindungsgemäße Verfahren das Extraktionsmittel N,N-Di-n-butylformamid vollständig und Ameisensäure weitgehend aus dem Abwasser entfernt werden kann.

## Patentansprüche

1. Verfahren zur Gewinnung von wasserfreier oder weitgehend wasserfreier Ameisensäure, bei dem man
(i) Methylformiat der Hydrolyse unterwirft,
(ii) vom erhaltenen Hydrolysegemisch Methanol sowie überschüssiges Methylformiat in einer Destillationseinrichtung abdestilliert und in den unteren Teil dieser Destillationseinrichtung Wasserdampf einleitet,
(iii) das Ameisensäure und Wasser enthaltende Sumpfprodukt der Destillation ii) in einer Flüssig-flüssig-Extraktion mit einem hauptsächlich die Ameisensäure aufnehmenden Extraktionsmittel extrahiert und eine Teilmenge der bei der Flüssig-flüssig-Extraktion entstehenden wäßrigen Phase als Abwasser aus dem Prozeß schleust,
(iv) die hierbei erhaltene Ameisensäure, Extraktionsmittel und eine Teilmenge des Wassers aufweisende Extraktphase einer Destillation unterwirft,
(v) das bei dieser Destillation erhaltene, Wasser und eine Teilmenge der Ameisensäure enthaltende Kopfprodukt in den unteren Teil der Destillationseinrichtung der Stufe ii) zurückführt,
(vi) das vorwiegend Extraktionsmittel und Ameisensäure enthaltende Sumpfprodukt der Destillationsstufe iv) destillativ in wasserfreie oder weitgehend wasserfreie Ameisensäure und das Extraktionsmittel auftrennt und
(vii) das die Stufe vi) verlassende Extraktionsmittel in den Verfahrensgang zurückführt,
**dadurch gekennzeichnet, daß** Wasserdampf, der in die für die Durchführung der Stufe ii) vorgesehene Destillationseinrichtung eingeleitet wird, vor dem Einleiten in diese Destillationseinrichtung, als Strippdampf für eine Abwasserstrippung des in Stufe iii) aus dem Prozeß ausgeschleusten Abwassers, eingesetzt wird.

2. Verfahren nach Anspruch 1, **dadurch gekennzeichnet, daß** als Extraktionsmittel ein Carbonsäureamid der allgemeinen Formel I 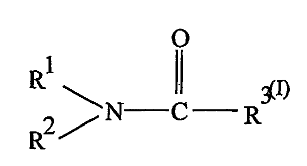 eingesetzt wird, in der die Reste R¹ und R² Alkyl-, Cycloalkyl-, Aryl- oder Aralkylgruppen bedeuten oder R¹ und R² gemeinsam zusammen mit dem N-Atom einen heterocyclischen 5- oder 6-Ring ausbilden und daß nur einer der Reste eine Arylgruppe ist und in der R³ für Wasserstoff oder eine C₁-C₄-Alkylgruppe steht.

3. Verfahren nach Anspruch 1 oder 2, **dadurch gekennzeichnet, daß** als Extraktionsmittel N,N-Di-n-butylformamid" N,N-Di-n-butylacetamid, N-Methyl-N-2-heptylformamid, N-n-Butyl-N-2-ethylhexylformamid, N-n-Butyl-N-cyclohexylformamid und/oder N-Ethylformanilid eingesetzt wird.

4. Verfahren nach einem der Ansprüche 1 bis 3, **dadurch gekennzeichnet, daß** die Schritte ii) und iv) in einer einzigen Destillationseinrichtung durchgeführt werden.

5. Verfahren nach einem der Ansprüche 1 bis 4, **dadurch gekennzeichnet, daß** der als Strippdampf eingesetzte Wasserdampf eine Temperatur von 110 bis 200°C, bevorzugt von 140 bis 180°C, aufweist.

6. Verwendung eines Wasserdampfstroms in dem Verfahren gemäß eines der Ansprüche 1 bis 5 zunächst als Strippdampfstrom für Abwasserstrippung und anschließend als Eduktstrom zur Bereitstellung von Wasser für die Hydrolyse von Methylformiat.

7. Vorrichtung zur Durchführung des Verfahrens gemäß eines der Ansprüche 1 bis 5, enthaltend,
α) einen Synthesereaktor (6),
β) einen Hydrolysereaktor (1),
χ) eine Destillationseinrichtung (2) zur Durchführung der Stufe ii),
δ) eine Destillationseinrichtung (4) zur Durchführung der Stufe iv),
ε) eine Extraktionseinrichtung (3),
φ) eine Destillationseinrichtung (5) zur Durchführung der Stufe vi)
und
γ) eine Abwasserstrippeinrichtung (10).

8. Vorrichtung nach Anspruch 7, **dadurch gekennzeichnet, daß** die Destillationseinrichtung (2) zur Durchführung der Stufe ii) und die Destillationseinrichtung (4) zur Durchführung der Stufe iv) in einer einzigen Destillationseinrichtung (7) angeordnet sind.

9. Vorrichtung nach Anspruch 7 oder 8, **dadurch gekennzeichnet, daß** als Abwasserstrippeinrichtung (10) eine Strippkolonne eingesetzt wird.

## Claims

1. A process for obtaining anhydrous or substantially anhydrous formic acid, in which
i) methyl formate is subjected to hydrolysis,
ii) methanol and excess methyl formate are distilled off from the resultant hydrolysis mixture in a distillation device, and steam is fed into the lower part of this distillation device,
iii) the bottom product from distillation ii), comprising formic acid and water, is extracted in a liquid-liquid extraction with an extractant which principally takes up the formic acid, and some of the aqueous phase formed in the liquid-liquid extraction is removed from the process as waste water,
iv) the resultant extract phase, comprising formic acid, extractant and some of the water, is subjected to distillation,
v) the top product obtained in this distillation, which comprises water and some of the formic acid, is fed back into the lower part of the distillation device in step ii),
vi) the bottom product from distillation step iv), which comprises predominantly extractant and formic acid, is separated by distillation into anhydrous or substantially anhydrous formic acid and the extractant, and
vii) the extractant leaving step vi) is fed back into the process,
which comprises employing steam introduced into the distillation device provided for carrying out step ii), before introduction into this distillation device, as stripping steam for waste-water stripping of the waste water removed from the process in step iii).

2. The process according to claim 1, wherein the extractant employed is a carboxamide of the general formula I 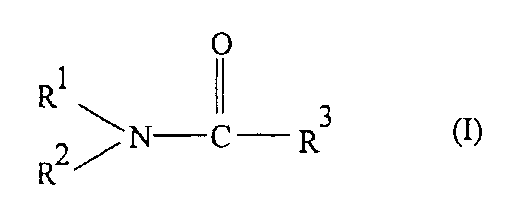 where the radicals R¹ and R² are alkyl, cycloalkyl, aryl or aralkyl groups, or R¹ and R² jointly, together with the N atom, form a heterocyclic 5- or 6-membered ring, and only one of the radicals is an aryl group, and where R³ is hydrogen or a C₁-C₄-alkyl group.

3. The process according to claim 1 or 2, wherein the extractant employed is N,N-di-n-butylformamide, N,N-di-n-butylacetamide, N-methyl-N-2-heptylformamide, N-n-butyl-N-2-ethylhexylformamide, N-n-butyl-N-cyclohexylformamide and/or N-ethylformanilide.

4. The process according to one of claims 1 to 3, wherein steps ii) and iv) are carried out in a single distillation device.

5. The process according to one of claims 1 to 4, wherein the steam employed as stripping steam has a temperature of from 110 to 200°C, preferably from 140 to 180°C.

6. The use of a steam stream in the process according to one of claims 1 to 5 firstly as stripping steam stream for waste-water stripping and subsequently as starting-material stream for providing water for the hydrolysis of methyl formate.

7. An apparatus for carrying out the process according to one of claims 1 to 5, comprising
α) a synthesis reactor, (6)
β) a hydrolysis reactor (1),
χ) a distillation device (2) for carrying out step ii),
δ) a distillation device (4) for carrying out step iv),
ε) an extraction device (3),
φ) a distillation device (5) for carrying out step vi), and
γ) a waste-water stripping device (10).

8. The apparatus according to claim 7, wherein the distillation device (2) for carrying out step ii) and the distillation device (4) for carrying out step iv) are arranged in a single distillation device (7).

9. The apparatus according to claim 7 or 8, wherein the waste-water stripping device (10) employed is a stripping column.

## Revendications

1. Procédé d'obtention d'acide formique anhydre ou largement anhydre, dans lequel :
(i) on soumet le méthylformiate à une hydrolyse,
(ii) on sépare le méthanol du mélange d'hydrolyse obtenu, ainsi que le méthylformiate en excès, par distillation dans une unité de distillation, et on introduit de la vapeur d'eau dans la partie inférieure de cette unité de distillation,
(iii) on extrait le produit de fond de colonne de la distillation (ii) contenant l'acide formique et l'eau lors d'une extraction liquide-liquide avec un agent d'extraction absorbant principalement l'acide formique et on élimine du procédé une quantité partielle de la phase aqueuse qui se forme lors de l'extraction liquide-liquide comme eau usée,
(iv) on soumet la phase d'extraction obtenue ici présentant l'acide formique, l'agent d'extraction et une quantité partielle de l'eau à une distillation,
(v) on recycle le produit de tête obtenu lors de la distillation contenant l'eau et une quantité partielle de l'acide formique dans la partie inférieure de l'unité de distillation de l'étape ii),
(vi) on sépare le produit de fond de colonne contenant principalement l'agent d'extraction et l'acide formique de l'étape de distillation iv) par distillation dans l'acide formique anhydre ou largement anhydre ainsi que l'agent d'extraction, et
(vii) on recycle l'agent d'extraction quittant l'étape vi) dans le processus du procédé,
**caractérisé en ce qu'**on utilise de la vapeur d'eau qui est introduite dans le dispositif de distillation prévu pour l'exécution de l'étape ii), avant l'introduction dans ce dispositif de distillation, en tant que vapeur d'entraînement pour une épuration de l'eau résiduaire évacuée du processus dans l'étape iii).

2. Procédé selon la revendication 1, **caractérisé en ce qu'**on utilise comme agent d'extraction un amide d'acide carboxylique de formule générale I : 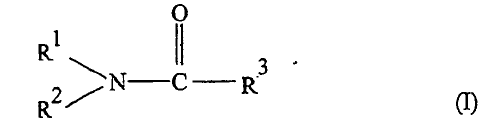 dans laquelle les radicaux R¹ et R² signifient des groupes alkyle, cycloalkyle, aryle ou aralkyle ou R¹ et R² forment ensemble avec l'atome de N un noyau hétérocyclique à 5 ou 6 chaînons et **en ce que** seul un des radicaux est un groupe aryle et dans laquelle R³ représente l'hydrogène ou un groupe alkyle en C₁-C₄.

3. Procédé selon la revendication 1 ou 2, **caractérisé en ce qu'**on utilise comme agent d'extraction le N,N-di-n-butylformamide, le N,N-di-n-butylacétamide, le N-méthyl-N-2-heptylformamide, le N-n-butyl-N-2-éthylhexylformamide, le N-n-butyl-N-cyclohexylformamide et/ou le N-éthylformanilide.

4. Procédé selon l'une des revendications 1 à 3, **caractérisé en ce que** les étapes ii) et iv) sont réalisées dans une seule et unique unité de distillation.

5. Procédé selon l'une quelconque des revendications 1 à 4, **caractérisé en ce que** la vapeur d'eau utilisée en tant que vapeur d'entraînement présente une température de 110 à 200°C, de préférence de 140 à 180 °C.

6. Utilisation d'un courant de vapeur d'eau dans le procédé selon l'une quelconque des revendications 1 à 5, d'abord en tant que courant de vapeur d'entraînement pour l'épuration d'eaux résiduaires et ensuite en tant que courant de départ pour la production de l'eau pour l'hydrolyse du formiate de méthyle.

7. Dispositif pour l'exécution du procédé selon l'une des revendications 1 à 5, contenant :
α) un réacteur de synthèse (6),
β) un réacteur d'hydrolyse (1),
χ) une unité de distillation (2) pour l'exécution de l'étape ii),
δ) une unité de distillation (4) pour l'exécution de l'étape iv),
ε) une unité d'extraction (3),
φ) une unité de distillation (5) pour l'exécution de l'étape vi), et
γ) un dispositif d'épuration d'eaux résiduaires (10).

8. Dispositif selon la revendication 7, **caractérisé en ce que** l'unité de distillation (2) est disposée pour l'exécution de l'étape ii), et l'unité de distillation (4) l'est pour l'exécution de l'étape iv) dans une seule et unique unité de distillation (7).

9. Dispositif selon la revendication 7 ou 8,
**caractérisé en ce qu'**on utilise comme dispositif d'épuration d'eaux résiduaires (10) une colonne d'extraction.
